# EUROPÄISCHE PATENTSCHRIFT

(11) **EP 0 460 143 B1**
(45) Veröffentlichungstag und Bekanntmachung des Hinweises auf die Patenterteilung: **24.05.1995**
(21) Anmeldenummer: 91900157.8
(22) Anmeldetag: 13.12.1990
(51) Int. Cl.: A61L 15/44, A61K 31/40

(54) **MITTEL ZUR TRANSDERMALEN APPLIKATION ENTHALTEND ROLIPRAM**
ROLIPRAM-CONTAINING COMPOSITION FOR TRANSDERMAL APPLICATION
COMPOSITION A APPLICATION TRANSCUTANEE CONTENANT DU ROLIPRAM

(30) Priorität: 27.12.1989 DE 3943385
(43) Veröffentlichungstag der Anmeldung: 11.12.1991
(73) Patentinhaber: SCHERING AKTIENGESELLSCHAFT, 13353 Berlin (DE)
(72) Erfinder: HÜMPEL, Michael, D-1000 Berlin 37 (DE); RIEDL, Jutta, D-1000 Berlin 19 (DE); HADGRAFT, Jonathan, Cardiff CF1 3XF (GB)
(86) Internationale Anmeldenummer: DE9000965
(87) Internationale Veröffentlichungsnummer: WO9109634

(56) Entgegenhaltungen:
- EP-A- 0 262 753
- US-A- 4 012 495
- US 4,012,495

## Beschreibung

Die Erfindung betrifft ein Mittel zur transdermalen Applikation, welches dadurch gekennzeichnet ist, daß es Rolipram und penetrationsverstärkende Mittel in einem transdermalen therapeutischen System enthält,
welches aus
a) einer undurchlässigen Deckschicht,
   einer an der Deckschicht haftenden, das Rolipram,
   und gewünschtenfalls penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
   einer abziehbaren Schutzschicht, oder
b) einer undurchlässigen Deckschicht,
   einem an oder in der Deckschicht befindlichen, das Rolipram und gewünschtenfalls penetrationsverstärkende Mittel enthaltenden Arzneimittelreservoir,
   für diese Komponenten durchlässigen Polymerschicht,
   einer durchlässigen gegebenenfalls penetrationsverstärkende Mittel enthaltende Hauthaftkleberschicht und
   einer abziehbaren Schutzschicht besteht.

Rolipram 4-(3-Cyclopentyloxy-4-methoxy-phenyl)-2-pyrrolidon, eine Verbindung der Formel 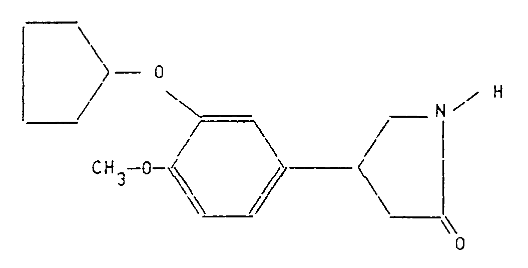
ist bekanntlich eine pharmakologisch wirksame Substanz mit einer starken antidepressiven Wirksamkeit (US-A 4,012,495).

Es wurde nun gefunden, daß Rolipram sehr gut zur Herstellung eines Mittels für die transdermale Applikation des Wirkstoffes verwendet werden kann.

Transdermal zu applizierende Arzneimittel haben bekanntlich den Vorzug, daß sie über einen längeren Zeitraum hin eine gleichmäßigere Freisetzung des Wirkstoffs ermöglichen, als dies in der Regel bei anders - wie zum Beispiel oral -zu applizierenden Mitteln möglich ist. Diese Eigenschaften lassen sich in einer Reihe von endokrinen Erkrankungen vorteilhaft ausnutzen. Für in Wasser schwer lösliche Wirkstoffe ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels Überraschenderweise möglich ist, eine therapeutisch ausreichende sehr gleichmäßige Penetrationsgeschwindigkeit des Roliprams durch die Haut zu erzielen. Außerdem gewährleisten sie eine sehr gleichmäßige Applikation mit eingestellter Dosierung des Wirkstoffes.

Geeignete transdermale therapeutische Systeme sind solche, die man üblicherweise zur percutanen Applikation von Wirkstoffen anwendet (Yie W. Chien: "Tansdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Reserch 1030 Hamilton Court Menlo Park CA 94025 (415) 328-2228).

Ein transdermales therapeutisches System gemäß Variante a stellt ein einfaches Matrixsystem dar. Es kann beispielsweise wie folgt hergestellt werden.

Eine Lösung oder Suspension von 1 bis 25 Gew. % Wirkstoff, 0-40 Gew. % eines penetrationsverstärkenden Mittels, 30-70 Gew. % eines medizinisch üblichen Klebers aufgefüllt mit einem geeigneten flüchtigen Lösungsmittels zu 100 Gew. % wird auf eine plane undurchlässige Deckschicht gestrichen und nach dem Trocknen mit einer abziehbaren Schutzschicht versehen.

Geeignete flüchtige Lösungsmittel sind beispielsweise niedere Alkohole mit maximal 4 Kohlenstoffatomen, Ketone oder niedere Carbonsäureester wie Ethanol, Isopropanol,Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Die Konzentration, in welcher der Wirkstoff- oder das Wirkstoffgemisch optimalerweise in dem Lösungsmittel gelöst oder suspendiert werden, beträgt für Rolipram üblicherweise 0,01 bis 25 Gewichtsprozent und muß im Einzelfalle mittels der dem Fachmann geläufigen Vorversuche, wie zum Beispiel der Bestimmung der erreichbaren Blutplasmakonzentrationen an Wirkstoff, bei ausgewählten erfindungsgemäßen Mitteln ermittelt werden.

Geeignete penetrationsverstärkende Mittel sind beispielsweise die oben genannten niederen Alkohole, ferner Alkohole wie 1,2-Propandiol oder Benzylalkohol, gesättigte und ungesättigte geradkettige oder verzweigte Fettalkohole mit 8 bis 18 Kohlenstoffatomen, wie Laurylalkohol oder Cetylalkohol, Kohlenwasserstoffe, wie Mineralöl, gesättigte und ungesättigte geradkettige oder verzweigte Fettsäuren mit 8 bis 18 Kohlenstoffatomen, wie Isostearinsäure oder Olsäure, Fettsäureester der allgemeinen Formel

CH₃-(CH₂)ₙ-COOR

worin
n eine Zahl von 2 bis 18 und
R einen geradkettigen oder verzweigten Alkylrest mit maximal 6 Kohlen stoffatomen bedeuten,
oder Dicarbonsäurediester der allgemeinen Formel

R'OCO(CH₂)ₘCOOR'

worin
m eine Zahl von 4 bis 8 und
R' jeweils einen Alkylrest mit maximal 6 Kohlenstoffatomen bedeuten, Fettsäureester, die sich für das erfindungsgemäße Mittel eignen, sind beispielsweise solche der Laurinsäure, Myristinsäure, Stearinsäure und Palmitinsäure,wie zum Beispiel die Methylester, Ethylester, Propylester, Isopropylester, Butylester, sec.-Butylester, Isobutylester dieser Säuren. Besonders bevorzugte Ester sind solche der Myristinsäure, wie deren Methylester und insbesondere deren Isopropylester. Geeeignete Dicarbonsäurediester sind beispielsweise beispielsweise das Diisopropyladipat, Diisobutyladipat und Diisopropylsebacat. Es bedarf keiner näheren Erläuterung, das auch Gemische dieser penetrationsverstärkenden Mittel zur Herstellung des erfindungsgemäßen Mittels geeignet sind.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete Lösungsmittel und penetrationsverstärkenden Mittel sind beispielsweise die bereits erwähnten Flüssigkeiten dieser Art. Als medizinisch übliche Kleber eignen sich beispielsweise Polyacrylate, Silicone, Polyurethane, sowie natürliche oder synthetische Kautschuke. Als weitere Matrixbildner kommen Celluloseether, Polyvinylverbindungen oder Silikate in Betracht.

Als Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 »m dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 »m. Die Abgabe der Wirkstoffe erfolgt vorzugsweise über eine Fläche von 5 bis 100 cm².

Ein transdermales therapeutisches System gemäß Variante b kann beispielsweise wie folgt hergestellt werden.

Eine undurchlässige Folie wird durch Wärme und/oder Zug so verformt, daß eine 0,1 bis 3 ml fassende Ausbuchtung entsteht. Diese wird mit einer wirkstoffhaltigen Lösung oder Suspension enthaltend 1-50 Gew. % Wirkstoff in einem penetrationsverstärkenden Mittel gefüllt. Die wirkstoffhaltige Lösung oder Suspension kann auch mit bis zu 10 Gew. % Matrixbildner verdickt sein.

Als Abdeckung des Reservoirs zur Haut hin dient eine aufgeschweißte oder aufgeklebte durchlässige Polymerschicht, auf welche eine durchlässige Hauthaftkleberschicht und eine abziehbare Schutzschicht angebracht wird.

Es können bei diesem System die oben erwähnten penetrationsverstärkenden Mittel angewendet werden. Als durchlässige Polymerschicht wird beispielsweise eine 20 bis 200 »m dicke Folie aus Celluloseestern, Celluloseethern, Siliconen oder Polyolefinverbindungen verwendet. Durch Varitation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

So lassen sich durch einfache Variation der verschiedenen Parameter transdermale therapeutische Systeme mit unterschiedlichen Freisetzungsraten des Wirkstoffes oder Wirkstoffgemisches herstellen, die zwecks Lagerung beispielsweise in Aluminiumfolien verpackt werden können.

Die nachfolgenden Ausführungsbeispiele dienen zur näheren Erläuterung der Erfindung. In ihnen wurden folgende Handelsprodukte verwendet:
Polyesterfolie von 0,074 mm Dicke (SkotchPak® 1009) des Herstellers 3M; Polypropylenfolie (Celgard® 2500) des Herstellers Celanese, Linerfolie SkotchPak® 1022 und 1360 vom Hersteller 3M; Transferkleber 9871 vom Hersteller 3M, Polyacrylester-Kleber vom Typ Sichello® J 6610-21 des Herstellers Henkel KG, Silikonklebstoff vom Typ X-7-2960 des Herstellers Dow Corning und Hydroxypropylcellulose des Typs Klucel® HXF des Herstellers Hercules.

### Beispiel

In 200 g einer 50 %igen Lösung von Polyacrylester-Klebstoff in Aceton/Benzin werden nacheinander
1,0 g Rolipram und
10,0 g Isopropylmyristat
unter Rühren gelöst bzw. suspendiert. Da die Klebstoffe trübe sind, läßt isch nicht klar entscheiden, ob eine klare Lösung vorliegt). Nach Entgasen des Ansatzes wird die Lösung/Suspension mittels einer Beschichtungsvorrichtung auf Polyesterfolie in der Weise aufgetragen, daß nach Entfernung der flüchtigen Lösemittel ein gleichmäßiger Film von 100 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

## Patentansprüche

1. Mittel zur transdermalen Applikation, welches dadurch gekennzeichnet ist, daß es Rolipram und penetrationsverstärkende Mittel in einem transdermalen therapeutischen System enthält,
welches aus
a) einer undurchlässigen Deckschicht,
einer an der Deckschicht haftenden, das Rolipram,
und gewünschtenfalls penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder
b) einer undurchlässigen Deckschicht,
einem an oder in der Deckschicht befindlichen, das Rolipram und gewünschtenfalls penetrationsverstärkende Mittel enthaltenden Arzneimittelreservoir,
für diese Komponenten durchlässigen Polymerschicht,
einer durchlässigen gegebenenfalls penetrationsverstärkende Mittel enthaltende Hauthaftkleberschicht und
einer abziehbaren Schutzschicht besteht.

## Claims

1. A preparation for transdermal administration that is characterised in that it comprises rolipram and penetration promotors in a transdermal therapeutic system which comprises
a) an impermeable cover layer,
a medicament layer which adheres to the cover layer, which comprises the rolipram and, if desired, penetration promotors and is permeable to those components, and which is self-adhesive or is covered or enclosed by a skin-adhesive layer which may likewise contain penetration promotors, and
a removable protective layer, or
b) an impermeable cover layer,
a medicament reservoir which is located on or in the cover layer and which comprises the rolipram and, if desired, penetration promotors,
a polymer layer permeable to those components,
a permeable skin-adhesive layer which optionally contains penetration promotors, and
a removable protective layer.

## Revendications

1. Composition ou produit à application transdermique ou percutanée, qui se caractérise en ce que la composition contient du "Rolipram" et un agent amplifiant la pénétration dans un système thérapeutique transdermique, qui consiste en :
a) une couche imperméable de couverture, une couche adhérant à la couche de couverture, contenant le "Rolipram" et éventuellement un agent amplifiant la pénétration, cette couche de médicament étant perméable pour ce constituant, et étant autoadhérente ou recouverte d'un adhésif collant à la peau ou entouré par un tel adhésif, qui peut éventuellement contenir un produit amplifiant la pénétration et une couche protectrice amovible, ou bien
b) une couche imperméable de couverture,
un réservoir de médicament se trouvant sur ou dans la couche de couverture et qui contient le "Rolipram" et éventuellement un produit amplifiant la pénétration,
une couche de polymère perméable à ces constituants,
une couche perméable, adhérante à la peau et contenant éventuellement un agent amplifiant la pénétration, et,
une couche protectrice amovible.
